# EUROPEAN PATENT APPLICATION

(11) **EP 1 929 947 A1**
(43) Date of publication of application: **11.06.2008**
(21) Application number: 06798497.1
(22) Date of filing: 26.09.2006
(51) Int. Cl.: A61B 5/117, G06T 1/00

(54) **FINGER VEIN PATTERN INPUT DEVICE**

(30) Priority: 30.09.2005 JP 2005285992
(71) Applicant: Hitachi Information & Control Solutions, Ltd., Hitachi-shi, Ibaraki 319-1293 (JP)
(72) Inventor: NAKAYAMA, Hisao, Hitachi-shi, Ibaraki 319-1293 (JP); NAKAI, Hiroto, Hitachi-shi, Ibaraki 319-1293 (JP); KIKUCHI, Kichiro, Hitachi-shi, Ibaraki 319-1293 (JP)
(74) Representative: Beetz & Partner
(86) International application number: PCT/JP2006/319656
(87) International publication number: WO 2007/037471

(57) **Abstract**

To provide a finger vein vein pattern input apparatus to be felt that one finger is put surely at a predetermined position during an authentication time, thereby a good convenience is attained.

A finger vein pattern input apparatus for a personal authentication is constituted having a main body, a finger put stand installed on the main body, an irradiation means installed in an interior portion of the main body and for light irradiating a finger put on the finger put stand, and an imaging means for taking an image the light irradiated finger through an imaging window formed the finger put stand. To the finger put stand, an irradiation window arranged on a penetrated hole provided on the finger put stand and for passing through a light from the irradiation means is provided, and a hand touch feeling section having a smooth surface of a hand touch by approaching the imaging window and the irradiation window and by distinguishing from a peripheral rough surface having grains.

## Description

### Technical Field

The present invention relates to a finger vein pattern input apparatus used for a personal authentication by utilizing pattern data of a finger vein.

### Background Technique

Japanese patent laid-open publication No. 2004-49705 describes a personal authentication apparatus having a case to which an insertion section for capable to insert a fingerprint of one finger toward a lower face is provided, a light source section for irradiating a transmission light against the finger from an upper face, an interference filter section having a transmission light performance to any waveform length, an imaging section for taking an image of the transmitted light which has transmitted to the interference filter section, and an image processing section of a vein imaging data of the finger which is outputted from the imaging section.

Japanese patent laid-open publication No. 2002-92616 describes an authentication apparatus in which a personal authentication is carried out by utilizing a vein pattern of a palm side of a hand in which a chip type near-infrared luminescence diode is used as a light source.

Japanese patent laid-open publication No. 2005-128936 describes a finger authentication apparatus having a finger put guide for setting a position of a finger, a light source section for irradiating a transmission light to the finger, an imaging section for taking an image of the transmission light, and an authentication section for carrying out an authentication processing of an image which has taken the image by the imaging section, further the finger put guide comprises an authentication object finger put guide section for setting a non-object of the finger of an authentication object and an authentication non-object finger put guide section for setting the finger of an authentication non-object.

In the above stated authentication object finger put guide section, a section corresponding to an optical opening portion, in which the finger of the authentication object is set and authenticated, opens and a root portion of the finger of the authentication object has a dent shape which is formed along to an outer shape thereof.

### Disclosure of the Invention

As an aim of safety management of an estate and information, an expectation for a personal authentication technique is heightened. In particularly, it has paid an attention in a vivisection authentication technique, which uses a part of a body of human as a key, there is a small possibility of a dishonest act such as loss and robbery etc. in comparison with management according to a conventional recitation number and a key. In an authentication technique using a blood vessel pattern of a finger, since it is not remind of a crime using a fingerprint and also since light is not irradiated directly to an eyeball such as an iris, there is no mental resistance feeling, further since a surface of a living body is not read but features of an inner portion of the living body are read, there has a merit in which forgery is difficult.

For the above stated conveniences, the authentication methods are used widely as the personal authentication from men to children and from adult humans to old humans. As stated in above, in the finger vein pattern input apparatus used for many humans, it is important that it can be felt by the human during the authentication time the authentication is carried out where the finger is put surely at a predetermined position and a secure feeling is held by the human.

For taking the above stated aspects under the consideration, an object of the present invention is to provide a finger vein pattern input apparatus where it can be felt by the human that the finger is put surely at a predetermined position and it can obtain a good convenience.

The present invention provides a finger vein pattern input apparatus for a personal authentication constituted having a main body, a finger put stand installed on the main body, an irradiation means installed in an interior portion of the main body and for light irradiating one finger put on the finger put stand, and an imaging means for taking an image the light irradiated finger through an imaging window which is formed the finger put stand, in the finger vein pattern input apparatus to the finger put stand, an irradiation window arranged on a penetrated hole provided on the finger put stand and for passing through a light from the irradiation means is provided, and a hand touch feeling section having a smooth surface of a hand touch by approaching the imaging window and the irradiation window and by distinguishing from a peripheral rough surface having grains (ruggedness, embossments).

The present invention provides a finger vein pattern input apparatus for a personal authentication constituted having a main body, a finger put stand installed on the main body, an irradiation means installed in an interior portion of the main body and for light irradiating one finger put on the finger put stand, and an imaging means for taking an image the light irradiated finger through an imaging window which is formed the finger put stand, in the finger vein pattern input apparatus to the finger put stand, an irradiation window arranged on a penetrated hole provided on the finger put stand and for passing through a light from the irradiation means is provided, and a hand touch feeling section having a different surface of a hand touch by approaching the imaging window and the irradiation window and by distinguishing from a peripheral surface.

A finger vein pattern input apparatus according to an embodiment of the present invention, in the above stated constitution, further it has a characteristic the hand touch feeling section is provided at both sides of the imaging window. Further, it has another characteristic the hand touch feeling section is arranged between the imaging window and the irradiation window. Further, it has a characteristic that the hand touch feeling section is provided at both sides of the imaging window, as forming the imaging window as a center, at one side one hand touch feeling section and one irradiation window arranged, at another side another hand touch feeling section and another irradiation window arranged with these order, the one side one hand touch feeling section and the one irradiation window and the another hand touch feeling section and the another irradiation window are in a row with one straight line.

Hereinafter, one embodiment of the present invention will be explained referring to the drawing.

According to the present invention, since the hand touch feeling section having a smooth surface of a hand touch is provided by approaching the imaging window and the irradiation window and by distinguishing from a peripheral rough surface having grains, to carry out the authentication when the finger is put on the finger put stand by crossing the imaging window, it is enable to feel that the finger is put surely at a predetermined position, accordingly the finger vein pattern input apparatus having a good convenience is provided.

### Brief Explanation of Drawing

Fig. 1 is an appearance view of a finger vein pattern input apparatus of an embodiment according to the present invention.
Fig. 2 is a plane view of Fig. 1.
Fig. 3 is a cross-sectional view of Fig. 1.
Fig. 4 is a view showing an assembly body of an infrared ray luminescence LED and a state display LED.
Fig. 5 is an image view showing a control of a finger position when a finger is put.

### Best Mode for Carrying out the Invention

### [Embodiment 1]

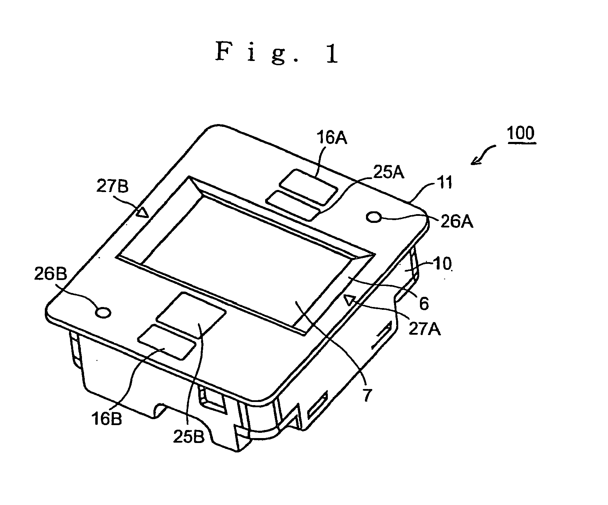

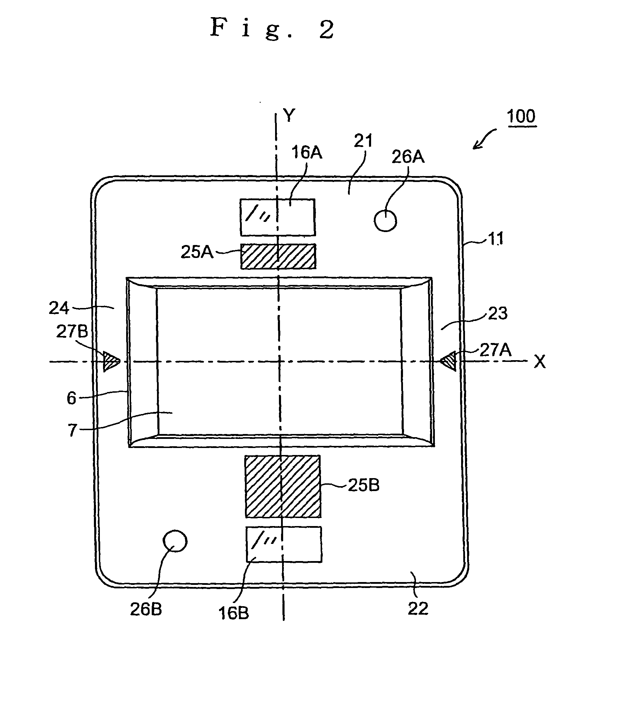

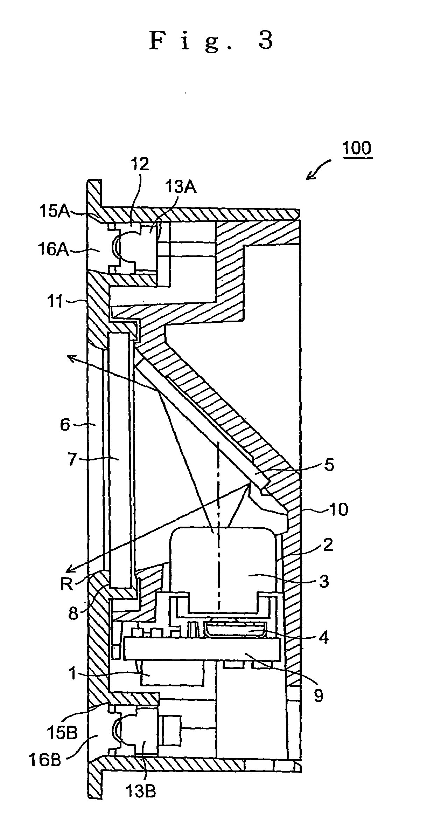

Fig. 1 is an appearance view of a finger vein pattern input apparatus 100 of an embodiment according to the present invention, Fig. 2 is a plane view thereof, and Fig. 3 is a cross-sectional view of a central portion of Fig. 2.

Firstly, a constitution of the finger vein pattern input apparatus 100 will be explained using Fig. 3.

To a main body 1 a finger put stand 11 is assembled and made as one body. Further, on an interior portion of the main body 10, a control module section (a control module means) 1, which is mounted on a camera substrate board 9 and in which CPU for carrying out an image processing is installed, and an imaging section (an imaging means) 2 are provided. The control module section 1 as already has known has a finger vein pattern extraction means for extracting a vein pattern from an image which has obtained by the imaging section 2, and an image execution means for comparing, namely colliding, an extracted finger vein pattern with a beforehand registered finger vein pattern.

The imaging section 2 comprises a near-infrared ray luminescence LED 13 (13A, 13B) as an irradiation means which is arranged in a space portion 12 formed in an upper portion (in the figure a vertical direction left side portion) of the main body 10 and a finger detecting means (not shown in figure).

To the finger put stand 11, a perforating portion 15 (15A, 15B) provided on the finger put stand 11 and an irradiation window 16 (16A, 16B) opposed with the near-infrared ray luminescence LED 13 are provided. The near-infrared ray luminescence LED 13 is arranged in the space portion 12 and the light does not leak except for the irradiation window 16. To a central portion of the main body 10, an optical axis refracting section 5 is comprised of a camera lens 3, a camera module 4 having CCD and a mirror. In the optical axis refracting section 5, the mirror is inclined and then a thickness of a whole apparatus is thinned and an optical path length of the imaging is lengthened and an image strain is deceased. An imaging opening portion 6 is formed largely at the central portion of the finger put stand 11 and to this imaging opening portion 6 an imaging window 7 is provided. To the imaging window 7 a smoke like material for covering the interior portion thereof is used. The infrared ray is transmitted to this material. The imaging opening portion 6 is formed with a shape in which a deep dent having a roundness R toward an outer portion and during the image taking time it makes not to contact the finger to the imaging window 7. By providing the above stated roundness R, it is possible to not be pressed neither crush the vein. Further, it is possible to form a good vein reflection, which positions a peripheral portion of the imaging opening portion. The imaging window 7 is installed vertically in a groove 8, which is provided on the imaging opening portion 6, as shown in figure.

A light projected through the camera lens 3 is bent by the mirror of the optical axis refracting portion 5 and passes through the imaging window 7 and the imaging opening portion 6 and the light is irradiated toward an outer portion as shown in an arrow mark.

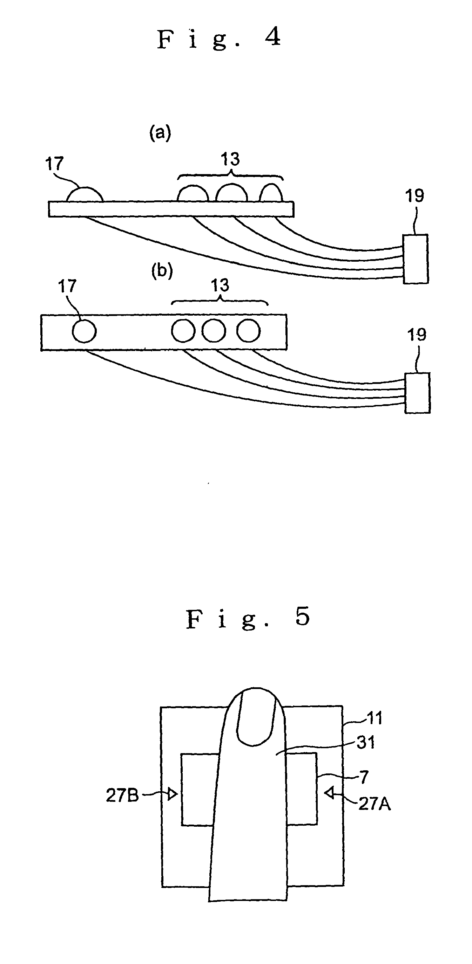

The near-infrared ray luminescence LED 13 and the state display LED 17 arranged in the space portion 12 as shown in Fig. 4. Fig. 4(a) is a side view and Fig. 4(b) is a plane view. In Fig. 4, plural near-infrared ray luminescence LED's 13 and one state display LED 17 are arranged on a substrate board 18. Each of components is connected to a control section 19. In this example, the state display LED presents green color when it turns on the light.

The lighting of the state display LED is as following.

| | | |
|---|---|---|
| A state: | finger put waiting | lighting |
| B state: | finger put confirmation image taking on | rapid blinking |
| C state: | authentication/registration OK | lighting |
| D state: | authentication/registration NG | slow blinking |

Fig. 2 shows an upper face portion of the finger put stand 11. As sated in above, the imaging opening portion 6 is provided on the central portion of the upper face portion of the finger put stand 11 and the imaging window 7 is provided on the imaging opening portion 6.

The peripheral portion of the imaging window 7 comprises four faces having flat like faces. A length toward a longitudinal direction of the finger put stand 11 in the figure is lengthened a little in comparison with a length toward a lateral direction. As stated shape in above, it is possible to put easily the finger on a surface and also it is possible to obtain surely an imaging picture.

On two parallel flat surfaces 21 and 21 on which the finger is put, the above stated irradiation windows 16A and 16B are provided separately from the imaging window 7. Between the irradiation windows 16A and 16B and the imaging window 7, namely by approaching respectively to the irradiation windows 16A and 16B and the imaging window 7, a hand touch feeling section 25 (25A, 26B) is provided. An area of the feeling section 25B is made large in comparison with an area of the feeling section 25A. Accordingly, it provides a difference between the areas according to whether a tip portion of the finger or a root portion. Each of the irradiation windows 16A and 16B and the hand touch feeling sections 25A and 26B forms a side length shape rectangular shape. It is possible to irradiate fully by a breadth of 8mm degree and it is possible to put the finger.

As stated in above, the hand touch feeling sections 25A and 26B are provided on both sides of the above stated imaging window and by forming the imaging window as a center at one side the hand touch feeling section 25A and the irradiation window 16A are arranged and at another side the hand touch feeling section 25B and the irradiation window 16B are arranged with this order, further to the one side hand touch feeling section 25A the one side irradiation window 16A and to the another side hand touch feeling section 25B the another side irradiation window 16B are arranged with one straight line Y.

The hand touch feeling of the peripheral flat surfaces 21 and 22 of the hand touch feeling section 25 (25A, 25B) is formed with a slippery feeling. Namely, the grains for expressing the unevenness of the peripheral flat surfaces 21 and 22 is made rough but the grains of the hand touch feeling section 25 are made thin. The hand touch feeling section 25 has enable to form entirely with a flat surface. As stated in above, the hand touch feeling section 25 having a hand touch smooth face is formed by distinguishing from the rough surface of the peripheral surface having the grains.

It is possible to make a constitution with a reversal feeling. Namely, it is possible to form with thin peripheral surface having the grains or to have a rough surface of the grains by distinguishing from one having the surface having no grain.

As stated in above, it is possible to form the hand touch feeling section 25 having a surface having a different feeling by distinguishing from the peripheral surface. It is possible to form directly the hand touch feeling section 25 on the flat surfaces 21 and 22, further it is possible to stick a plastic plate (a different material) having the above stated characteristics. Further, it is possible to delete the surfaces of the flat surfaces 21 and 22. The flat surfaces 21 and 22 are provided separately at a side portion of the imaging window 7 by corresponding the state display window 26 (26A, 26B) with the state display LED 17.

On the side portion other flat surfaces 23 and 24, a front and back direction discrepancy pressing means 27 (27A, 27B) is provided at one straight line X to a perpendicular direction with the above stated straight line Y and a surface thereof is formed at the same to the surface of the hand touch feeling section 25.

Fig. 5 is a view shows that by forming the imaging window 7 as a center, the right and left direction discrepancy of the finger 31 is carried out using the hand touch feeling section 25A and the irradiation window 16A and the hand touch feeling section 25B and the irradiation window 16B, these arranged in one straight line. When the finger 31 will separate from the hand touch feeling sections 25A and 25B, the finger 31 contacts the peripheral flat surfaces 21 and 22 having the different hand touch, thereby the finger will be felt a foreign feeling. Accordingly, since the finger 31 is put on the central portion, it is possible to prevent the right and left direction discrepancy of the finger. When the finger 31 is put, the front and back direction discrepancy pressing means 27A and 27B of the finger makes the feeling of the before and back direction discrepancy or carry out the view-recognition and have a role of an action for put the finger at a suitable position. For example, by making the right and left direction discrepancy pressing means 27A and 27B as a mark, by suiting a first joint of the finger, it is possible to put the finger at a suitable position toward a front and back direction.

## Claims

1. In a finger vein pattern input apparatus for a personal authentication constituted having a main body, a finger put stand installed on said main body, an irradiation means installed in an interior portion of said main body and for light irradiating one finger put on said finger put stand, and an imaging means for taking an image said light irradiated finger through an imaging window which is formed said finger put stand, the finger vein pattern input apparatus **characterized in that**, to said finger put stand, an irradiation window arranged on a penetrated hole provided on said finger put stand and for passing through a light from said irradiation means is provided, and a hand touch feeling section having a smooth surface of a hand touch by approaching said imaging window and said irradiation window and by distinguishing from a peripheral rough surface having grains.

2. A finger vein pattern input apparatus according to claim 1, **characterized in that** said hand touch feeling section is provided at both sides of said imaging window.

3. A finger vein pattern input apparatus according to claim 1, **characterized in that** said hand touch feeling section is arranged between said imaging window and said irradiation window.

4. A finger vein pattern input apparatus according to claim 3, **characterized in that** said hand touch feeling section is provided at both sides of said imaging window, as forming said imaging window as a center, at one side one hand touch feeling section and one irradiation window arranged, at another side another hand touch feeling section and another irradiation window arranged with these order, said one side one hand touch feeling section and said one irradiation window and said another hand touch feeling section and said another irradiation window are in a row with one straight line.

5. A finger vein pattern input apparatus according to claim 4, **characterized in that** a finger front and back direction discrepancy pressing means is provided to be arranged a perpendicular direction against said one straight line and is enable to be felt on one straight line at both sides of said imaging window or to be view-recognized.

6. In a finger vein pattern input apparatus for a personal authentication constituted having a main body, a finger put stand installed on said main body, an irradiation means installed in an interior portion of said main body and for light irradiating a finger put on said finger put stand, and an imaging means for taking an image said light irradiated finger through an imaging window which is formed said finger put stand, the finger vein pattern input apparatus **characterized in that**, to said finger put stand, an irradiation window arranged on a penetrated hole provided on said finger put stand and for passing through a light from said irradiation means is provided, and a hand touch feeling section having a different surface of a hand touch by approaching said imaging window and said irradiation window and by distinguishing from a peripheral surface.
